# EUROPEAN PATENT SPECIFICATION

(11) **EP 1 207 881 B1**
(45) Date of publication and mention of the grant of the patent: **01.04.2015**
(21) Application number: 00951523.0
(22) Date of filing: 16.08.2000
(51) Int. Cl.: A61K 31/4406, A61K 47/06, A61K 47/02, A61P 35/00, A61P 37/00, A61P 1/00, A61P 3/10, A61P 5/00, A61P 17/00

(54) **Pharmaceutical agent comprising a benzamide derivative as active ingredient**
Benzamidderivate enthaltende pharmazeutische Mittel
Agent pharmaceutique comprenant un derivé benzamide comme principe actif

(30) Priority: 16.08.1999 JP 22955199
(43) Date of publication of application: 29.05.2002
(73) Proprietor: Bayer Intellectual Property GmbH, 40789 Monheim am Rhein (DE)
(72) Inventor: Suzuki, Tsuneji, Chiba 297-0017 (JP); Ando, Tomoyuki, Chiba 297-0017 (JP); Ishibashi, Masahiko, 1900-1, Togo, Mobara-shi, Chiba 297-0017 (JP); Sakabe, Masahiro, 1900-1, Togo, Mobara-shi, Chiba 297-0017 (JP); Sakai, Ikuo, 1900-1, Togo, Mobara-shi, Chiba 297-0017 (JP)
(74) Representative: Dörries, Hans Ulrich
(86) International application number: PCT/EP2000/008011
(87) International publication number: WO 2001/012193

(56) References cited:
- EP-A- 0 847 992

## Description

### Field of Invention

The present invention relates to a pharmaceutical composition and in particular to a pharmaceutical formulations comprising as an active ingredient a benzamide derivative or a pharmaceutically acceptable salt thereof, that is useful as a pharmaceutical agent, in particular an anticancer agent.

### Background Art

Benzamide derivatives or pharmaceutically acceptable salts thereof according to the present invention have an ability of inhibiting histone deacetylating enzymes and of inducing differentiation, and are useful as therapeutic or ameliorating agents for diseases that are involved in cellular growth such as malignant tumors, autoimmune diseases, skin diseases, infections, blood vessel diseases, allergic diseases, gastrointestinal disorders, hormonal diseases, diabetes mellitus, and the like, enhancers of the effect of gene therapy, or immunosuppressants. In particular, they are effective as anti-tumor agents and are effective against hematopoietic organ tumors and solid tumors (Japanese Unexamined Patent Publication (Kokai) No. 10-152462).

However, though the benzamide derivatives and pharmaceutically acceptable salts thereof of the present invention are stable per se, they become unstable and decompose markedly over time when combined with additives such as light silicic acid anhydride, lactose, corn starch, carboxymethyl cellulose, magnesium alminate metasilicate, titanium oxide, polyethylene glycols and polysorbates that are commonly used in order to produce dosage forms suitable for oral, percutaneous, or tissue administration.

Furthermore, when they are formulated into tablets by the wet granulation, the most common granulation method of preparing solid formulations, they become further unstable and yield, in large quantities, decomposed products different from simple hydrolyzates, resulting in pharmaceutical formulations in which the ratio of an active ingredient is as low as about 0.001 to 25%, which noticeably decompose, and therefore which are unsuitable as pharmaceutical solid formulations to be provided as medical drugs. Also, pharmaceutical formulations that employ ingredients commonly used for liquids such as polysorbates, polyethylene glycols, and glycerin were unstable. Thus it was difficult to use, as medical drugs, pharmaceutical formulations that contain a benzamide derivative or a pharmaceutically acceptable salt thereof at about 0.001 to 25% as an active ingredient.

### Disclosure of Invention

The present invention is intended to enhance the stability of compositions containing as an active ingredient a pharmaceutically useful benzamide derivative or a pharmaceutically acceptable salt and to effectively use them as a pharmaceutical formulation.

In order to solve the above problems, intensive research was conducted on the effects of temperature, humidity, and physicochemical properties on the solutions, powders, and solid shaped products to which a benzamide derivative or a pharmaceutically acceptable salt thereof has been added. As a result, the inventors have found that the problem of instability of an active ingredient can be solved and stable and excellent pharmaceutical formulations can be produced by using selectively, among the additives commonly used for pharmaceutical formulations, those additives that do not easily induce decomposition of benzamide derivatives, adding an organic acid salt, an amino compound and an inorganic basic substance, and the like as a stabilizer, producing using the dry granulation or adjusting pH in the range of 4 to 12, preferably in the range of pH 7 to 11, and thereby have completed the present invention.

Thus, the present invention relates to
[1] a pharmaceutical formulation comprising a benzamide derivative represented by the formula (1): 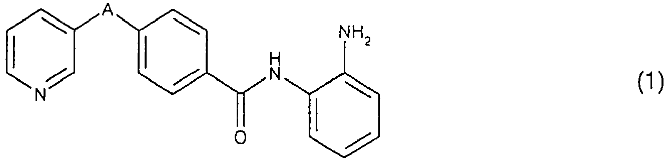 wherein A represents a structure shown by any one of the formula (2): 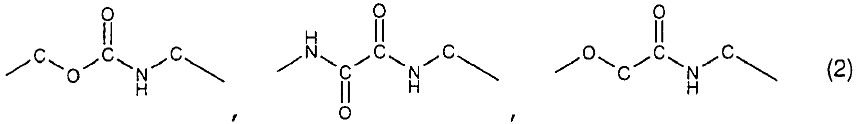 or a pharmaceutically acceptable salt thereof,
   an excipient selected from the group consisting of lactose, lactose anhydride, D-mannitol, corn starch, and crystalline cellulose,
   a lubricant selected from the group consisting of magnesium stearate, calcium stearate, stearic acid, and talc,
   a disintegrant selected from the group consisting of partly pregelatinized starch, carmellose calcium and carboxymethylstarch sodium,
   and one or more than one selected from the group consisting of an amino compound and an inorganic basic substance,
   wherein the amino compound is one or more than one selected from the group consisting of tris(hydroxymethyl)aminomethane, monoethanolamine, diethanolamine, triethanolamine, diisopropanolamine, triisopropanolamine, dihydroxyaluminum aminoacetate, arginine, creatinine, sodium glutamate, glycine, L-arginine L-glutamate, and carbachol,
   and the inorganic basic substance is one or more than one selected from the group consisting of sodium carbonate, potassium carbonate, ammonium carbonate, sodium bicarbonate, potassium bicarbonate, sodium hydroxide, disodium phosphate, and ammonia.
[2] as a preferred embodiment the pharmaceutical formulation of the above [1] wherein said benzamide derivative is represented by the formula (3); 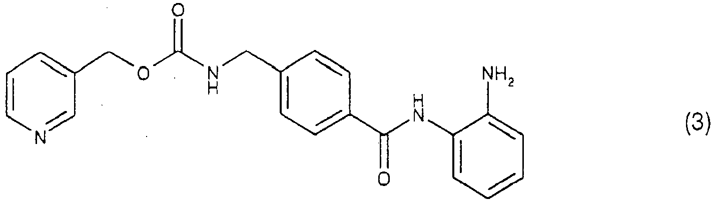
[3] as a preferred embodiment the pharmaceutical formulation of the above [1] or [2] wherein said excipient is D-mannitol;
   the lubricant is selected from the group consisting of magnesium stearate and talc, the disintegrant is selected from the group consisting of partly pregelatinized starch, carmellose calcium and carboxymethylstarch sodium,
   the amino compound is one or more than one selected from the group consisting of tris(hydroxymethyl)aminomethane, monoethanolamine, diethanolamine, triethanolamine, diisopropanolamine, triisopropanolamine, dihydroxyaluminum aminoacetate, arginine, creatinine, sodium glutamate, glycine, L-arginine L-glutamate, and carbachol,
   and the inorganic basic substance is one or more than one selected from the group consisting of sodium carbonate, potassium carbonate, ammonium carbonate, sodium bicarbonate, potassium bicarbonate, sodium hydroxide, disodium phosphate, and ammonia.
[4] In another preferred embodiment, the present invention relates to a pharmaceutical formulation comprising a benzamide derivative represented by the formula (1): 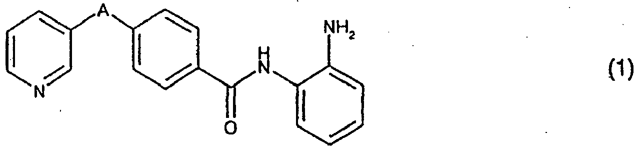 wherein A represents a structure shown by any one of the formula (2): 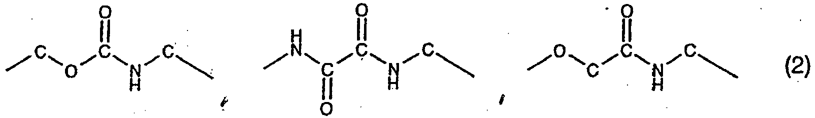 or a pharmaceutically acceptable salt thereof,
   one or more than one solvent(s) selected from the group consisting of propylene glycol, dimethylacetamide, and polyethylene glycol,
   and one or more than one selected from the group consisting of an organic acid salt, an amino compound and an inorganic basic substance,
   wherein the organic acid salt is one or more than one selected from the group consisting of monosodium fumarate, sodium alginate, sodium dehydroacetate, sodium erythorbate, and trisodium citrate,
   the amino compound is one or more than one selected from the group consisting of tris(hydroxymethyl)aminomethane, monoethanolamine, diethanolamine, triethanolamine, diisopropanolamine, triisopropanolamine, dihydroxyaluminum aminoacetate, arginine, creatinine, sodium glutamate, glycine, L-arginine L-glutamate, and carbachol,
   and the inorganic basic substance is one or more than one selected from the group consisting of sodium carbonate, potassium carbonate, ammonium carbonate, sodium bicarbonate, potassium bicarbonate, sodium hydroxide, disodium phosphate, and ammonia,
   wherein the pH of the formulation is adjusted in the range of 7 to 11.
[5] as a particularly preferred embodiment the pharmaceutical formulation of the above [4], wherein said solvent is polyethylene glycol 400.
[6] as a preferred embodiment the pharmaceutical formulation of the above [4] or [5] wherein said benzamide derivative is represented by the formula (3): 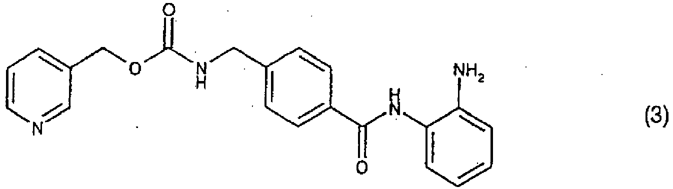
[7] as a preferred embodiment the pharmaceutical formulation of the above [1] to [3] wherein
   the formulation is a solid formulation which comprises granules prepared by a dry granulation method; and
[8] as a preferred embodiment, the pharmaceutical formulation of any one of the above [4] to [6] wherein the formulation is a liquid formulation and pH is adjusted within the range of 7 to 11.

### Embodiment for Carrying Out the Invention

The present invention will now be explained in further detail below.

The pharmaceutical formulations as used herein generally mean those that have been produced by formulating one or more additives with an active ingredient or active ingredients and that have been formulated into shapes suitable for use in various dosage forms of medical drugs.

According to the present invention, solid formulations, in particular powders, can be produced by adding to the active ingredient one or more than one additives that do not easily induce decomposition by using a method conventionally used by a person skilled in the art. Examples of additives that do not easily induce decomposition include: D-mannitol as an excipient; partly pregelatinized starch, carboxymethylstarch sodium, and carmellose calcium as a disintegrant; hydroxypropyl cellulose as a binder; magnesium stearate and talc as a lubricant; and hydroxypropyl methyl cellulose as a coating agent. One or more than one of them can be used.

According to the present invention, solid formulations, in particular granules, tablets, and capsules can be produced by a dry granulation method in which additives that do not easily induce decomposition are added to the active ingredient, mixed in a shaker such as a granulator and a V-type mixer, compression-molded by a roller compactor after the mixture in a shaker, and further crushed by a power mill thereby to form granules.

Furthermore, more stable granules, tablets, and capsules can be obtained by adding to the active ingredient one or more than one selected from the group consisting of an amino compound such as tris(hydroxymethyl)aminomethane, monoethanolamine, diethanolamine, triethanolamine, diisopropanolamine, triisopropanolamine, dihydroxyaluminum aminoacetate, arginine, creatinine, sodium glutamate, glycine, L-arginine L-glutamate, and carbachol; and an inorganic basic substance such as sodium carbonate, potassium carbonate, lithium carbonate, strontium carbonate; ammonium carbonate, sodium bicarbonate, potassium bicarbonate, lithium bicarbonate, strontium bicarbonate, sodium hydroxide, disodium phosphate, and ammonia, and by granulating in the dry granulation method.

In accordance with the present invention, stable liquids, syrups, injections, emulsions, suspensions, suppositories, soft capsules whose contents are liquid, or hard capsules whose contents are liquid and the like can be produced by dissolving an active ingredient into solvents that do not easily induce the decomposition of the active ingredient such as propylene glycol, polyethylene glycol and dimethylacetamide, by using a method conventionally used by a person skilled in the art.

More stable liquids, syrups, injections, emulsions, suspensions, suppositories, soft capsules whose contents are liquid, or hard capsules whose contents are liquid and the like can be produced by dissolving in a solvent one or more than one ingredients, selected from the group consisting of an organic acid salt such as monosodium fumarate, sodium alginate, sodium dehydroacetate, sodium erythorbate and trisodium citrate; an amine compound such as tris(hydroxymethyl)aminomethane, monoethanolamine, diethanolamine, triethanolamine, diisopropanolamine, triisopropanolamine, dihydroxyaluminum aminoacetate, arginine, creatinine, sodium glutamate, glycine, L-arginine L-glutamate, and carbachol; an inorganic basic substance such as ammonium carbonate, disodium phosphate, sodium carbonate, potassium carbonate, lithium carbonate, strontium carbonate, sodium bicarbonate, potassium bicarbonate, lithium bicarbonate, strontium bicarbonate, sodium hydroxide and ammonia, and by adjusting pH in the range of 7 to 11 with an acid or a base.

As used herein, acids or bases mean organic bases, inorganic bases, organic acids, or inorganic acids that can be used as medical drugs. Organic bases mean tris(hydroxymethyl)aminomethane, monoethanolamine, diethanolamine, triethanolamine, diisopropanolamine, triisopropanolamine, arginine, and the like. Inorganic bases mean sodium hydroxide, ammonium water, potassium bicarbonate, potassium carbonate, sodium bicarbonate, sodium carbonate, and the like. Organic acids mean citric acid, succinic acid, acetic acid, tartaric acid, lactic acid, and the like. Inorganic acids mean hydrochloric acid, sulfuric acid, phosphoric acid and the like.

In order to produce lyophilized formulations, according to the present invention, an active ingredient is mixed with a conventionally known solvent such as one or more than one solvent selected from the group consisting of purified water, macrogol, propylene glycol, polysorbate and dimethylacetamide; to a resulting composition are further added one or more than one additive selected from the group consisting of sugars; gelatin; dextrin; an organic acid salt such as monosodium fumarate, sodium alginate, sodium glutamate, sodium dehydroacetate, sodium erythorbate, trisodium citrate, and arginine-glutamate; an amine compound such as tris(hydroxymethyl)aminomethane, ammonia water, monoethanolamine, diethanolamine, triethanolamine, diisopropanolamine, triisopropanolamine, dihydroxyaluminum aminoacetate, arginine, creatinine, glycine, and carbachol; an inorganic basic substance such as ammonium carbonate, disodium phosphate, sodium carbonate, sodium bicarbonate and potassium bicarbonate; and then pH of the resulting composition is adjusted to 7 to 11, as desired, with an acid or a base, and the composition is freeze-dried under a reduced pressure.

The pharmaceutical formulations of the present invention can be administered by any method depending on various dosage forms, the age and sex of the patient, the severity of disease, and other conditions. For example, tablets, pills, liquids, syrups, suspensions, emulsions, granules, and capsules may be orally administered, injections may be intravenously administered either singly or in an admixture with a conventional supplement such as glucose and an amino acid, and, as needed, may be administered singly intramuscularly, subcutaneously, or intraperitoneally. Lyophilized formulations reconstituted with solvent such as saline and purified water may be administered intravenously singly or in an admixture with a conventional supplement such as glucose, an amino acid and the like, and, as needed, may be administered singly intramuscularly, subcutaneously, or intraperitoneally. Suppositories may be directly administered intrarectally.

Dosages of the pharmaceutical formulations of the present invention are selected as appropriate depending on the method of administration, the age and sex of the patient, the severity of disease, and the like. Generally the daily dosage of an active ingredient compound is preferably in the range of about 0.0001 to 100 mg/kg, and for pharmaceutical formulations in the unit dosage form an active ingredient compound is preferably included at a range of about 0.001 to 1,000 mg.

Benzamide derivatives, active ingredients of the present invention, or pharmaceutically acceptable salts thereof can be produced by a method described in, for example, Japanese Unexamined Patent Publication (Kokai) No. 10-152462.

Medical drugs as used herein mean, in addition to anticancer agents, agents for the treatment and/or amelioration of autoimmune diseases, skin diseases, infections, diseases of blood vessels, allergic diseases, gastrointestinal disorders, hormonal diseases, diabetes mellitus, and the like, enhancers of the effect of gene therapy, or immunosuppressants.

### Examples

The present invention will now be explained in more detail with reference to the following compound, N-(2-aminophenyl)-4-[N-(pyridine-3-yl)methoxycarbonyl]aminomethyl benzamide (compound 1), in Examples and Reference Examples. It is to be noted, however, that the present invention is not limited by these examples in any way.

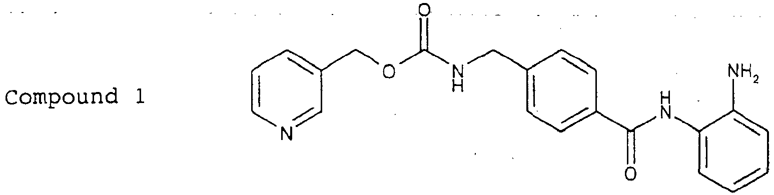

### Example 1.

Compound 1 (1 g) was mixed with 1 g each of D-mannitol, partly pregelatinized starch, carmellose calcium, hydroxypropyl cellulose, hydroxypropyl methyl cellulose, magnesium stearate, and talc to prepare a powder formulation. Similarly, lactose, corn starch, crystalline cellulose, carmellose, light-weight silicic acid anhydride, magnesium aluminum metasilicate, and titanium oxide were mixed to prepare a comparative control sample. After these formulations were stored at an air-tight condition at 60°C for 4 weeks and at an open condition at 40°C and at a relative humidity of 75% for 3 months, they were subjected to HPLC analysis. The percentage (%) of degradation products relative to the active ingredient was shown in Table 1. The powder formulation prepared by mixing 1:1 with D-mannitol, partially gelatinized starch, carmellose calcium, hydroxypropyl cellulose, hydroxypropyl methyl cellulose, magnesium stearate or talc was stable.

**Table (1): Stability of various powders**

| | Storage condition | |
|---|---|---|
| Additive | 60°C air-tight | 40°C 75% RH open |
| | 4 weeks | 3 months |
| Comparative control sample | | |
| None | 0.18 | 0.19 |
| Lactose | 0.55 | 0.44 |
| Corn starch | 0.39 | 0.34 |
| Crystalline cellulose | 0.25 | 0.61 |
| Carmellose | 0.43 | 0.41 |
| Light-weight silicic acid anhydride | 5.87 | 10.01 |
| Magnesium aluminum metasilicate | 17.94 | 5.45 |
| Titanium oxide | 1.75 | 0.82 |
| Example | | |
| D-mannitol | 0.21 | 0.21 |
| Partially gelatinized starch | 0.21 | 0.34 |
| Carmellose calcium | 0.30 | 0.21 |
| Hydroxypropyl cellulose | 0.20 | 0.20 |
| Magnesium stearate | 0.22 | 0.20 |
| Hydroxypropyl methyl cellulose | 0.27 | 0.21 |
| Talc | 0.36 | 0.23 |

Figures in the table represent the total amount (%) of the degradation products produced by the decomposition of compound 1.

### Example 2.

Pharmaceutical formulations a, b, c, d, e, and f shown in Table (2) were prepared in the following procedure. Thus, compound 1 and D-mannitol divided into 1/8, 2/8, and 5/8 of the prescribed amount were serially added under mixing using a granulator to prepare homogeneous powders. Furthermore, 1/2 of the prescribed amount of magnesium stearate was added thereto and was mixed in a V-shaped mixer for 20 minutes, compression-molded by a roller compactor, and further crushed by a power mill to prepare granules. Subsequently, carboxymethyl starch sodium of the prescribed amount and 1/2 of the prescribed amount of magnesium stearate were added and mixed in a V-shaped mixer, made into tablets by tabletting machine to obtain samples a, b, c, d, e, and f.

**Table (2): Formulation for tablets (unit: mg)**

| Ingredient/number | Sample of the present invention | | | | | |
|---|---|---|---|---|---|---|
| | a | b | c | d | e | f |
| Active ingredient | 5.0 | 1.0 | 1.0 | 1.0 | 1.0 | 1.0 |
| D-mannitol | 56.0 | 60.0 | 60.0 | 60.0 | 60.0 | 60.0 |
| Carboxymethyl starchsodium | 3.3 | 3.3 | 3.3 | 3.3 | 3.3 | 3.3 |
| Magnesium stearate | 0.7 | 0.7 | 0.7 | 0.7 | 0.7 | 0.7 |
| Tris(hydroxymethyl) aminomethane | - | - | 0.5 | - | - | - |
| Potassium bicarbonate | - | - | - | 0.5 | - | - |
| Sodium carbonate | - | - | - | - | 0.5 | - |
| Potassium carbonate | - | - | - | - | - | 0.5 |
| Total | 65.0 | 65.0 | 65.5 | 65.5 | 65.5 | 65.5 |

### Reference Example 1.

D-mannitol, partially gelatinized starch, carmellose calcium, magnesium stearate, hydroxypropyl cellulose, polyvinylpyrrolidone K30, or the like which is comparatively stable when mixed with Compound 1 was granulated according to the formulation shown in Table (3) by the wet granulation method and made into tablets by tabletting machine to obtain samples g to i.

**Table (3): Formulation for tablets (unit: mg)**

| Ingredient/number | Sample | | |
|---|---|---|---|
| | g | h | i |
| Active ingredient | 1.0 | 1.0 | 1.0 |
| D-mannitol | 40.6 | 40.6 | 40.6 |
| Partially gelatinized starch | 17.4 | 17.4 | 17.4 |
| Hydroxypropyl cellulose | 2.0 | 2.0 | - |
| Polyvinylpyrrolidone | - | - | 2.0 |
| Carmellose calcium | 3.3 | - | 3.3 |
| Magnesium stearate | 0.7 | 0.7 | 0.7 |
| Total | 65.0 | 65.0 | 65.0 |

The percentage (%) of degradation products of compound 1 is shown in Table 4, when samples obtained in Example 2 and Reference Example 1 were stored at an air-tight condition at 60°C for 4 weeks and at an air-tight condition at 80°C for 3 days, and then were subjected to HPLC analysis. The pharmaceutical formulations containing 1 mg of an active ingredient obtained by the wet granulation method shown in Reference Example 1 were unstable as they produced degradation products other than the hydrolyzates, while the samples of the present invention shown in Example 2, both 5.0 mg- and 1.0 mg-containing formulations, were stable as the production of degradation products remained low.

**Table (4): Stability of tablets containing compound 1**

| Sample | | Storage condition | | |
|---|---|---|---|---|
| | | Content (mg) | 60°C air-tight | 80°C air-tight |
| | | | 4 weeks (%) | 3 days (%) |
| The invention sample | a | 5.0 | 0.4 | 0.4 |
| | b | 1.0 | 1.0 | 1.3 |
| | c | 1.0 | 0.7 | 0.5 |
| | d | 1.0 | - | 0.4 |
| | e | 1.0 | - | 0.4 |
| | f | 1.0 | - | 0.4 |
| Reference example | g | 1.0 | 4.1 | 3.0 |
| | h | 1.0 | 4.5 | 2.1 |
| | i | 1.0 | 5.8 | 5.3 |

Figures in the table represent the total amount (%) of the degradation products produced by the decomposition of compound 1.

### Example 3.

Compound 1 was dissolved to a concentration of 20 mg/ml in propylene glycol or dimethylacetamide to prepare liquid formulations. As comparative control samples, compound 1 was dissolved to a concentration of 20 mg/ml in polysorbate 80 or polyethylene glycol 400. Table (5) shows the percentage (%) of degradation products of compound 1 when these formulations were stored at an air-tight condition at 80°C for 3 days. They exhibited good stability when dissolved in propylene glycol or dimethylacetamide.

**Table (5): Stability when dissolved in various solvents**

| Additive | Amount of degradation products (%) |
|---|---|
| Polysorbate 80 | 18.1 |
| Polyethylene glycol 400 | 41.4 |
| Dimethylacetamide | 4.1 |
| Propylene glycol | 3.6 |

Figures in the table represent the total amount (%) of the degradation products produced by the decomposition of compound 1.

### Example 4.

Compound 1 was dissolved to a concentration of 20 mg/ml in polyethylene glycol 400 to prepare a liquid formulation, which was set as a comparative control sample. To the comparative control sample was added each additive at a concentration of 0.05 M to prepare the liquid formulation of the present invention. Table (6) shows the percentage (%) of degradation products of the active ingredients when these formulations and the comparative control samples were stored at an air-tight condition at 80°C for 3 days. Stability was enhanced in the samples to which an organic acid salt, an amino compound, or an inorganic basic substance of the present invention was added.

**Table (6): Stability of liquid formulations in which each additive was blended at 0.05 M to compound 1 at 20 mg/ml polyethylene glycol 400 (Storage condition: 80°C, air tight, Storage period: 3 days)**

| | Additive | Amount of degradation products | pH |
|---|---|---|---|
| | | (%) | |
| Comparative control sample | None | 41.4 | 5.3 |
| The invention sample | Sodium fumarate | 21.6 | 7.0 |
| | Sodium alginate | 23.7 | 6.7 |
| | Sodium dehydroacetate | 13.0 | 8.6 |
| | Sodium erythorbate | 13.2 | 7.3 |
| | Trisodium citrate | 28.2 | 8.0 |
| | Tris(hydroxymethyl)aminomethane | 2.9 | 10.1 |
| | Monoethanolamine | 4.3 | 11.5 |
| | Diethanolamine | 3.9 | 11.7 |
| | Triethanolamine | 9.6 | 9.4 |
| | Diisopropanolamine | 4.7 | 9.9 |
| | Triisopropanolamine | 16.5 | 8.3 |
| | Dihydroxyaluminum aminoacetate | 7.3 | 6.4 |
| | L-arginine | 10.6 | 11.5 |
| | Creatinine | 18.6 | 7.0 |
| | Sodium glutamate | 23.1 | - |
| | Glycine | 26.7 | - |
| | L-arginine L-glutamate | 29.4 | 6.5 |
| | Carbachol | 32.3 | 5.4 |
| | Ammonium carbonate | 3.6 | 10.7 |
| | Disodium phosphate | 10.8 | 7.6 |
| | Sodium carbonate | 16.8 | 10.1 |
| | Sodium bicarbonate | 25.0 | 6.5 |
| | Potassium bicarbonate | 15.5 | 7.0 |
| | Ammonia | 4.6 | 11.7 |

Figures in the table represent the total amount (%) of the degradation products produced by the decomposition of compound 1.

### Example 5.

To compound 1 dissolved at a concentration of 20 mg/ml in polyethylene glycol 400 was added an equal volume of 0.1 M tris(hydroxymethyl)aminomethane buffer of which pH is varied with hydrochloric acid or sodium hydroxide to prepare a liquid formulation of compound 1 at 10 mg/ml. Table 7 shows the percentage of the degradation products of the active ingredient when these formulations were stored at an air-tight condition at 80°C for 3 days. Samples of the present invention of which pH was adjusted in the range of 7 to 11 exhibited good stability.

**Table (7): Stability of 10 mg/ml polyethylene glycol 400 solution of compound 1 when pH was varied (storage condition: 80°C, air-tight, storage period: 3 days)**

| pH | Amount of degradation products (%) |
|---|---|
| 3.8 | 98.6 |
| 13.2 | 48.8 |
| 7.3 | 11.9 |
| 7.7 | 8.2 |
| 8.5 | 5.8 |
| 9.5 | 5.6 |
| 10.1 | 4.4 |

Figures in the table represent the total amount (%) of the degradation products produced by the decomposition of compound 1.

### Example 6.

Compound 1 was dissolved at a concentration of 20 mg/ml in polyethylene glycol 400, and sodium hydroxide was added thereto so that the final various concentrations can be from 0 mM to 10 mM to prepare liquid formulations. Table 8 shows the percentage of the degradation products of the active ingredient when these formulations were stored at each pH of these formulations and at an air-tight condition at 80°C for 1 day or 7 days. Samples of the present invention of which pH was adjusted in the range of about 7 to 11 exhibited good stability.

**Table (8): Relationship between pH and stability of liquid formulations in which compound 1 was dissolved at 20 mg/ml in polyethylene glycol 400, and then sodium hydroxide was added thereto (storage condition: 80°C, air-tight, storage period: 1 day or 7 days)**

| Concentration of sodium hydroxide | pH | Amount of degradation products (%) | |
|---|---|---|---|
| (mM) | | 80°C - 1 day | 80°C - 7 days |
| 0 | 5.3 | 16.0 | 63.7 |
| 0.01 | 5.9 | 14.1 | 60.6 |
| 0.1 | 6.1 | 14.3 | 56.0 |
| 1.0 | 7.3 | 9.7 | 33.7 |
| 2.0 | 8.9 | 4.6 | 12.4 |
| 3.0 | 9.4 | 5.0 | 9.8 |
| 4.0 | 10.4 | 6.0 | 9.7 |
| 5.0 | 10.8 | 9.7 | 11.4 |
| 10.0 | 13.1 | 71.5 | 90.6 |

Figures in the table represent the total amount (%) of the degradation products produced by the decomposition of compound 1.

### Industrial Applicability

Pharmaceutical formulations that produce little degradation products and that are stable enough to be used as medical drugs can be obtained by mixing a pharmaceutically useful benzamide derivatives or a pharmaceutically acceptable salt thereof with additives that do not easily produce degradation products, blending an organic acid salt, an amine compound, or an inorganic basic substance, producing solid formulations by the dry granulation method, and further adjusting the pH of the liquid formulations to 7 to 11.

## Claims

1. A pharmaceutical formulation comprising a benzamide derivative represented by the formula (1): 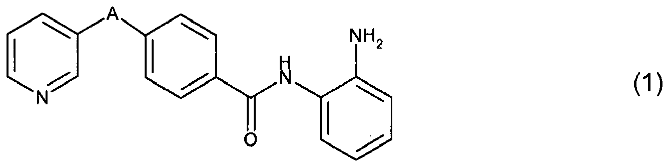 wherein A represents a structure shown by any one of the formula (2): 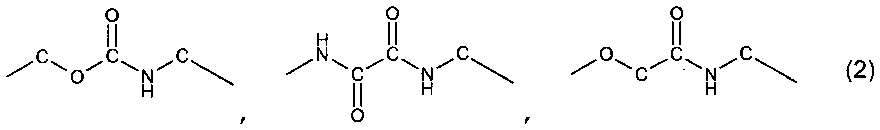 or a pharmaceutically acceptable salt thereof,
an excipient selected from the group consisting of lactose, lactose anhydride, D-mannitol, corn starch, and crystalline cellulose,
a lubricant selected from the group consisting of magnesium stearate, calcium stearate, stearic acid, and talc,
a disintegrant selected from the group consisting of partly pregelatinized starch, carmellose calcium and carboxymethylstarch sodium,
and one or more than one selected from the group consisting of an amino compound and an inorganic basic substance,
wherein the amino compound is one or more than one selected from the group consisting of tris(hydroxymethyl)aminomethane, monoethanolamine, diethanolamine, triethanolamine, diisopropanolamine, triisopropanolamine, dihydroxyaluminum aminoacetate, arginine, creatinine, sodium glutamate, glycine, L-arginine L-glutamate, and carbachol,
and the inorganic basic substance is one or more than one selected from the group consisting of sodium carbonate, potassium carbonate, ammonium carbonate, sodium bicarbonate, potassium bicarbonate, sodium hydroxide, disodium phosphate, and ammonia.

2. The pharmaceutical formulation according to claim 1,
wherein the excipient is D-mannitol,
the lubricant is selected from the group consisting of magnesium stearate and talc,
the disintegrant is selected from the group consisting of partly pregelatinized starch, carmellose calcium and carboxymethylstarch sodium,
the amino compound is one or more than one selected from the group consisting of tris(hydroxymethyl)aminomethane, monoethanolamine, diethanolamine, triethanolamine, diisopropanolamine, triisopropanolamine, dihydroxyaluminum aminoacetate, arginine, creatinine, sodium glutamate, glycine, L-arginine L-glutamate, and carbachol,
and the inorganic basic substance is one or more than one selected from the group consisting of sodium carbonate, potassium carbonate, ammonium carbonate, sodium bicarbonate, potassium bicarbonate, sodium hydroxide, disodium phosphate, and ammonia.

3. A pharmaceutical formulation comprising a benzamide derivative represented by the formula (1): 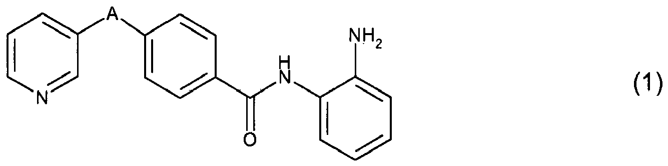 wherein A represents a structure shown by any one of the formula (2): 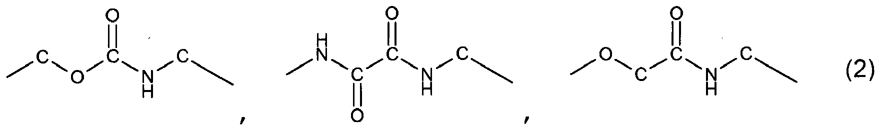 or a pharmaceutically acceptable salt thereof,
one or more than one solvent(s) selected from the group consisting of propylene glycol, dimethylacetamide, and polyethylene glycol,
and one or more than one selected from the group consisting of an organic acid salt, an amino compound and an inorganic basic substance,
wherein the organic acid salt is one or more than one selected from the group consisting of monosodium fumarate, sodium alginate, sodium dehydroacetate, sodium erythorbate, and trisodium citrate,
the amino compound is one or more than one selected from the group consisting of tris(hydroxymethyl)aminomethane, monoethanolamine, diethanolamine, triethanolamine, diisopropanolamine, triisopropanolamine, dihydroxyaluminum aminoacetate, arginine, creatinine, sodium glutamate, glycine, L-arginine L-glutamate, and carbachol,
and the inorganic basic substance is one or more than one selected from the group consisting of sodium carbonate, potassium carbonate, ammonium carbonate, sodium bicarbonate, potassium bicarbonate, sodium hydroxide, disodium phosphate, and ammonia,
wherein the pH of the formulation is adjusted in the range of 7 to 11.

4. The pharmaceutical composition according to any one of claims 1 to 3, wherein said benzamide derivative is represented by the formula (3):

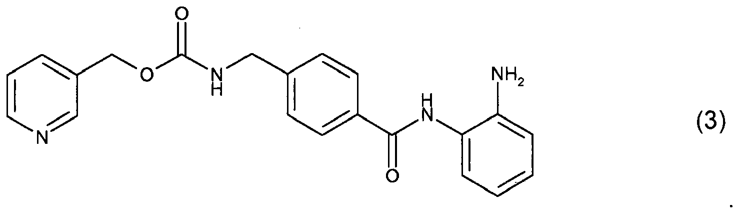

5. The pharmaceutical formulation according to claim 3 comprising
(i) a benzamide derivative represented by the formula (3): 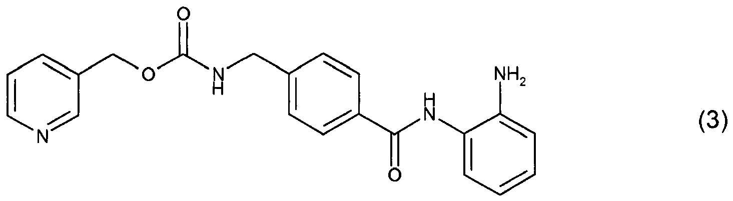
(ii) polyethylene glycol 400 as solvent; and
(iii) one selected from the group consisting of monosodium fumarate, sodium alginate, sodium dehydroacetate, sodium erythorbate, trisodium citrate, tris(hydroxymethyl)aminomethane, monoethanolamine, diethanolamine, triethanolamine, diisopropanolamine, triisopropanolamine, dihydroxyaluminium aminoacetate, L-arginine, creatinine, sodium glutamate, glycine, L-arginine L-glutamate, carbachol, sodium carbonate, ammonium carbonate, sodium bicarbonate, potassium bicarbonate, sodium hydroxide, disodium phosphate and ammonia.

## Patentansprüche

1. Eine pharmazeutische Formulierung umfassend ein Benzamid-Derivat, dargestellt durch Formel (1): 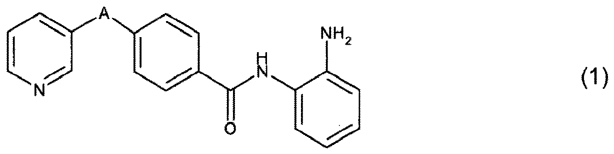 wobei A eine Struktur darstellt, die durch eine der Strukturen der Formel (2) abgebildet wird: 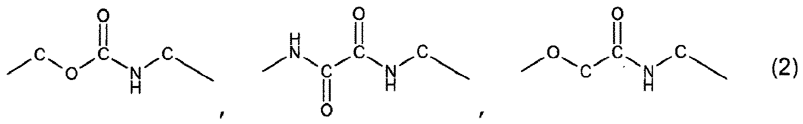 oder ein pharmazeutisch zulässiges Salz davon,
ein Hilfsstoff, gewählt aus der Gruppe bestehend aus Lactose, Lactose-Anhydrid, D-Mannitol, Maisstärke und kristalliner Zellulose,
ein Schmiermittel, gewählt aus der Gruppe bestehend aus Magnesiumstearat, Calciumstearat, Stearinsäure und Talk,
ein Sprengmittel, gewählt aus der Gruppe bestehend aus teilweise gequollener Stärke, Carmellose-Calcium und Natriumcarboxymethylstärke
und eine oder mehr als eine gewählt aus der Gruppe bestehend aus einer Aminoverbindung und einer anorganischen basischen Substanz,
wobei die Aminoverbindung eine oder mehr als eine ist, ausgewählt aus der Gruppe bestehend aus Tris(hydroxymethyl)aminomethan, Monoethanolamin, Diethanolamin, Triethanolamin, Diisopropanolamin, Triisopropanolamin,
Dihydroxyaluminiumaminoacetat, Arginin, Kreatinin, Natriumglutamat, Glycin, L-Arginin, L-Glutamat und Carbachol
und die anorganische basische Substanz ist eine oder mehr als eine gewählt aus der Gruppe bestehend aus Natriumcarbonat, Kaliumcarbonat, Ammoniumcarbonat, Natriumbicarbonat, Kaliumbicarbonat, Natriumhydroxid, Dinatriumphosphat und Ammoniak,

2. Die pharmazeutische Formulierung nach Anspruch 1,
wobei der Hilfsstoff D-Mannitol ist,
das Schmiermittel aus der Gruppe bestehend aus Magnesiumstearat und Talkgewählt ist,
das Sprengmittel aus der Gruppe bestehend aus teilweise gequollener Stärke, Carmellose-Calcium und Natriumcarboxymethylstärke gewählt ist,
die Aminoverbindung eine oder mehr als eine, gewählt aus der Gruppe bestehend aus Tris(hydroxymethyl)aminomethan, Monoethanolamin, Diethanolamin, Triethanolamin, Diisopropanolamin, Triisopropanolamin, Dihydroxyaluminiumaminoacetat, Arginin, Kreatinin, Natriumglutamat, Glycin, L-Arginin, L-Glutamat und Carbachol ist,
und die anorganische basische Substanz eine oder mehr als eine, gewählt aus der Gruppe bestehend aus Natriumcarbonat, Kaliumcarbonat, Ammoniumcarbonat, Natriumbicarbonat, Kaliumbicarbonat, Natriumhydroxid, Dinatriumphosphat und Ammoniak ist,

3. Eine pharmazeutische Formulierung umfassend ein Benzamid-Derivat dargestellt durch Formel (1): 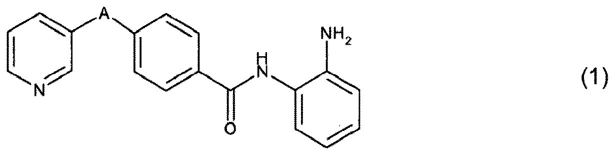 wobei A durch eine Struktur darstellt, die durch eine der Strukturen der Formel (2) abgebildet wird: 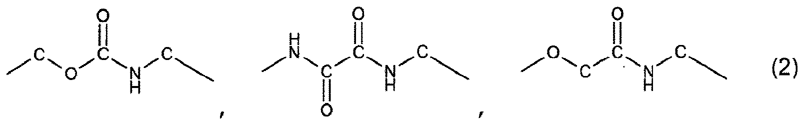 oder ein pharmazeutisch zulässiges Salz davon,
ein oder mehr als ein Lösungsmittel gewählt aus der Gruppe bestehend aus Propylenglycol, Dimethylacetamid und Polyethylenglycol
und einer oder mehr als einer gewählt aus der Gruppe bestehend aus einem Salz einer organischen Säure, einer Aminoverbindung und einer anorganischen basischen Substanz,
wobei das Salz einer organischen Säure eine oder mehr als eine gewählt aus der Gruppe bestehend aus Mononatriumfumarat, Natriumalginat, Natriumdehydroacetat, Natriumerythorbat und Trinatriumcitrat,
die Aminoverbindung ist eine oder mehr als eine gewählt aus der Gruppe bestehend aus Tris(hydroxymethyl)aminomethan, Monoethanolamin, Diethanolamin, Triethanolamin, Diisopropanolamin, Triisopropanolamin, Dihydroxyaluminiumaminoacetat, Arginin, Kreatinin, Natriumglutamat, Glycin, L-Arginin, L-Glutamat und Carbachol und die anorganische basische Substanz ist eine oder mehr als eine gewählt aus der Gruppe bestehend aus Natriumcarbonat, Kaliumcarbonat, Ammoniumcarbonat, Natriumbicarbonat, Kaliumbicarbonat, Natriumhydroxid, Dinatriumphosphat und Ammoniak,
wobei der pH der Formulierung im Bereich von 7 bis 11 eingestellt wird.

4. Die pharmazeutische Verbindung nach den Ansprüchen 1 bis 3, wobei das genannte Benzamid-Derivat durch Formel (3) dargestellt wird:

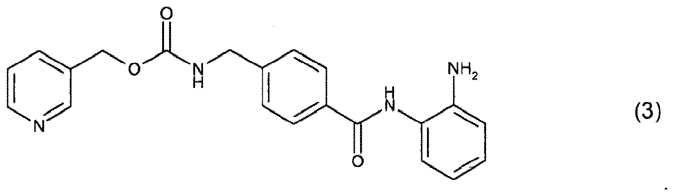

5. Die pharmazeutische Formulierung gemäß Anspruch 3 umfassend
(i) ein Benzamid-Derivat dargestellt durch Formel (3): 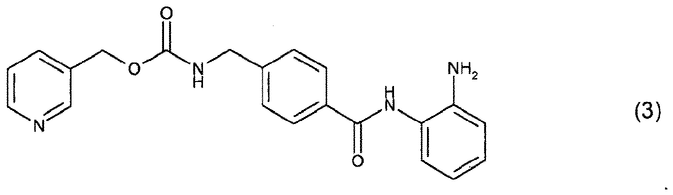
(ii) Polyethylenglycol 400 als Lösungsmittel; und
(iii) eine gewählt aus der Gruppe bestehend aus Mononatriumfumarat, Natriumalginat, Natriumdehydroacetat,
Natriumerythorbat und Trinatriumcitrat, Tris(hydroxymethyl)aminomethan, Monoethanolamin, Diethanolamin, Triethanolamin, Diisopropanolamin, Triisopropanolamin, Dihydroxyaluminiumaminoacetat, L-Arginin, Kreatinin, Natriumglutamat, Glycin, L-Arginin L-Glutamat, Carbachol, Natriumcarbonat, Ammoniumcarbonat, Natriumbicarbonat, Kaliumbicarbonat, Natriumhydroxid, Dinatriumphosphat und Ammoniak.

## Revendications

1. Formulation pharmaceutique comprenant un dérivé de benzamide représenté par la formule (1) : 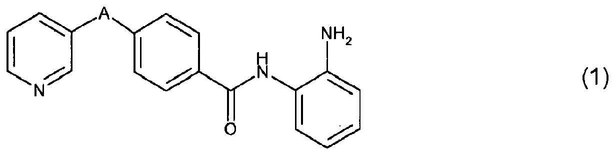 dans laquelle A représente une structure représentée par l'une quelconque des formules (2) : 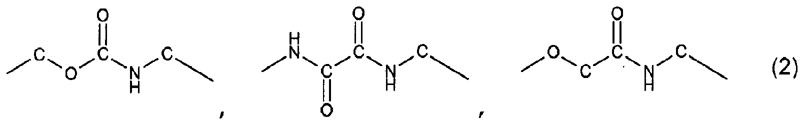 ou un de ses sels pharmaceutiquement acceptables,
un excipient choisi dans le groupe consistant en le lactose, le lactose anhydre, le D-mannitol, l'amidon de maïs et la cellulose cristalline,
un lubrifiant choisi dans le groupe consistant en le stéarate de magnésium, le stéarate de calcium, l'acide stéarique et le talc,
un agent de délitement choisi dans le groupe consistant en l'amidon partiellement prégélatinisé, le croscarmellose sodique et le carboxyméthylamidon sodique,
et un ou plusieurs agents choisis dans le groupe consistant en un composé à fonction amino et une substance basique inorganique,
où le composé à fonction amino est un ou plusieurs composés choisis dans le groupe consistant en le tris(hydroxyméthyl)-aminométhane, la monoéthanolamine, la diéthanolamine, la triéthanolamine, la diisopropanolamine, la triisopropanol-amine, l'aminoacétate de dihydroxyaluminium, l'arginine, la créatinine, le glutamate de sodium, la glycine, le L-glutamate de L-arginine et le carbachol,
et la substance basique inorganique est une ou plusieurs substances choisies dans le groupe consistant en le carbonate de sodium, le carbonate de potassium, le carbonate d'ammonium, le bicarbonate de sodium, le bicarbonate de potassium, l'hydroxyde de sodium, le phosphate disodique et l'ammoniac.

2. Formulation pharmaceutique suivant la revendication 1,
dans laquelle l'excipient est le D-mannitol,
le lubrifiant est choisi dans le groupe consistant en le stéarate de magnésium et le talc, l'agent de délitement est choisi dans le groupe consistant en l'amidon partiellement prégélatinisé, le croscarmellose sodique et le carboxyméthylamidon sodique,
le composé à fonction amino est un ou plusieurs composés choisis dans le groupe consistant en le tris(hydroxyméthyl)-aminométhane, la monoéthanolamine, la diéthanolamine, la triéthanolamine, la diisopropanolamine, la triisopropanol-amine, l'aminoacétate de dihydroxyaluminium, l'arginine, la créatinine, le glutamate de sodium, la glycine, le L-glutamate de L-argine et le carbachol,
et la substance basique inorganique est une ou plusieurs substances choisies dans le groupe consistant en le carbonate de sodium, le carbonate de potassium, le carbonate d'ammonium, le bicarbonate de sodium, le bicarbonate de potassium, l'hydroxyde de sodium, le phosphate disodique et l'ammoniac.

3. Formulation pharmaceutique comprenant un dérivé de benzamide représenté par la formule (1) : 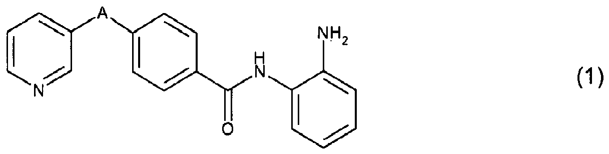 dans laquelle A représente une structure représentée par l'une quelconque des formules (2) : 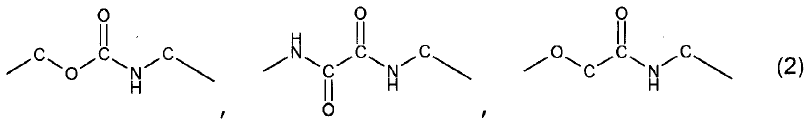 ou un de ses sels pharmaceutiquement acceptables,
un ou plusieurs solvants choisis dans le groupe consistant en le propylèneglycol, le diméthylacétamide et le polyéthylène-glycol,
et un ou plusieurs agents choisis dans le groupe consistant en un sel d'acide organique, un composé à fonction amino et une substance basique inorganique,
où le sel d'acide organique est un ou plusieurs sels choisis dans le groupe consistant en le fumarate monosodique, l'alginate de sodium, le dihydroacétate de sodium, l'érythorbate de sodium et le citrate trisodique,
le composé à fonction amino est un ou plusieurs composés choisis dans le groupe consistant en le tris(hydroxyméthyl)-aminométhane, la monoéthanolamine, la diéthanolamine, la triéthanolamine, la diisopropanolamine, la triisopropanol-amine, l'aminoacétate de dihydroxyaluminium, l'arginine, la créatinine, le glutamate de sodium, la glycine, le L-arginine, le L-glutamate et le carbachol,
et la substance basique inorganique est une ou plusieurs substances choisies dans le groupe consistant en le carbonate de sodium, le carbonate de potassium, le carbonate d'ammonium, le bicarbonate de sodium, le bicarbonate de potassium, l'hydroxyde de sodium, le phosphate disodique et l'ammoniac,
le pH de la formulation étant ajusté dans l'intervalle de 7 à 11.

4. Composition pharmaceutique suivant l'une quelconque des revendications 1 à 3, dans laquelle ledit dérivé de benzamide est représenté par la formule (3) :

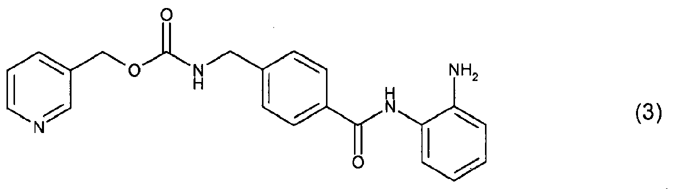

5. Formulation pharmaceutique suivant la revendication 3, comprenant
(i) un dérivé de benzamide représenté par la formule (3) : 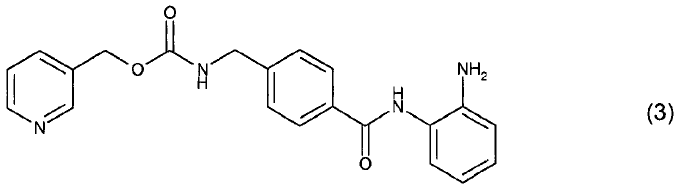
(ii) du polyéthylèneglycol 400 comme solvant ; et
(iii) un composé choisi dans le groupe consistant en le fumarate monosodique, l'alginate de sodium, le dihydro-acétate de sodium, l'érythorbate de sodium et le citrate trisodique, le tris(hydroxyméthyl)-aminométhane, la monoéthanolamine, la diéthanolamine, la triéthanolamine, la diisopropanolamine, la triisopropanolamine, l'aminoacétate de dihydroxy-aluminium, l'arginine, la créatinine, le glutamate de sodium, la glycine, le L-arginine, le L-glutamate, le carbachol, le carbonate de sodium, le carbonate d'ammonium, le bicarbonate de sodium, le bicarbonate de potassium, l'hydroxyde de sodium, le phosphate disodique et l'ammoniac.
